# EUROPÄISCHE PATENTSCHRIFT

(11) **EP 0 658 104 B1**
(45) Veröffentlichungstag und Bekanntmachung des Hinweises auf die Patenterteilung: **23.10.1996**
(21) Anmeldenummer: 93919102.9
(22) Anmeldetag: 19.08.1993
(51) Int. Cl.: A61K 9/20

(54) **LÄNGLICHE, TEILBARE TABLETTE**
DIVISIBLE OBLONG TABLET
COMPRIME OBLONG SECABLE

(30) Priorität: 01.09.1992 DE 4229085
(43) Veröffentlichungstag der Anmeldung: 21.06.1995
(73) Patentinhaber: BOEHRINGER MANNHEIM GMBH, 68298 Mannheim (DE)
(72) Erfinder: DEMMER, Fritz, D-69493 Hirschberg (DE); GABEL, Rolf-Dieter, D-68723 Schwetzingen (DE); PREIS, Walter, D-67433 Neustadt (DE)
(86) Internationale Anmeldenummer: EP9302218
(87) Internationale Veröffentlichungsnummer: WO9405264

(56) Entgegenhaltungen:
- WO-A-89/05624
- US-A- 4 824 677

## Beschreibung

Die Erfindung betrifft eine längliche, teilbare Tablette, insbesondere für pharmazeutische Anwendungen, deren Tablettenunterseite und Tablettenoberseite an den beiden Enden je einen derart heraustretenden Bereich aufweisen, daß beim Aufliegen einer der genannten Seiten auf einer flachen Unterlage die heraustretenden Bereiche, aber nicht der Bereich dazwischen, die Unterlage berührt. Die Tablettenunterseite und die Tablettenoberseite sind, wie in W. A. Rietschel "Die Tablette", Editio Cantor KG, Seite 58, 1966 angegeben, definiert.

Bei der Entwicklung von festen Arzneien wird nach solchen Darreichungsformen gesucht, welche, wie z.B. kleine runde Tabletten, besonders wirtschaftlich herstellbar sind. Im praktischen Gebrauch wurden bei runden Tabletten einige Nachteile festgestellt. Das Hauptproblem insbesondere für ältere Menschen stellt die Teilbarkeit solcher Tabletten dar. In der Anfangsphase der Behandlung wird vielfach die Hälfte der Dosierung gefordert (einschleichende Dosierung). Die Teilung ist deshalb schwierig, weil der Bruchspalt sehr fein ist und leicht übersehen wird oder die Patienten nicht genau wissen, wie sie Tablette teilen sollen. Das Ergebnis sind zwei unterschiedlich große oder sogar viele kleine Bruchstücke. Zum Teilen braucht man außerdem beide Hände. Gerade bei ältern Menschen kommt hinzu, daß sie Schwierigkeiten haben, kleine und leichte Tabletten von einer Unterlage aufzunehmen. Es kann deshalb sein, daß der Patient den Arzt nach einer leichter handhabbaren Darreichungsform fragt. Diese Nachteile sind nur unwesentlich geringer, wenn die Tablette statt einer runden eine Oblong-Form hat.

Es hat nicht an Versuchen gefehlt, das Problem der Teilbarkeit beispielsweise mit kleinen Apparaten, wie Kleinguillotinen, zu lösen. Solche Apparate sind im Handel erhältlich. Die Bedienung ist aber aufwendig und das Gerät muß jeweils dem Durchmesser der Tablette angepaßt werden. Es wurde auch eine sog. Knicktablette entwickelt. Sie hat die Form eines Winkels - ist also nicht symmetrisch - und bleibt daher nicht in der gewünschten Stellung auf einer Unterlage stehen. Um die Knicktablette zu teilen, sind zwei Hände erforderlich. Ein zusätzlicher Nachteil dieser Tablette besteht darin, daß sie auf Verpackungsmaschinen nicht verblistert werden kann. Eine geringe mechanische Belastung führt zum Bruch der Tablette. Eine weitere Ausführungsform einer teilbaren Tablette ist die von der Firma SKB vertriebene Tiltab ^{R}, die sich gut von der Unterlage aufnehmen läßt. Dies ist deshalb der Fall, weil diese Tablette, wenn sie auf eine Unterlage gelegt wird, - aufgrund ihrer Geometrie - immer auf einer Seite in die Höhe steht. Wegen ihrer Länge (14mm) und ihrer ovalen Form ist die Tablette relativ leicht zu teilen. Gerade die ovale Form ist aber von großem Nachteil auf der Verpackungsmaschine, weil eine ovale Tablette auf den Zuführungsschienen verkantet. Es kommt hinzu, daß die Anfertigung der Preßwerkzeuge aufwendig und teuer ist.

Es sind darüber hinaus Tabletten bekannt, die so ausgebildet sind, daß sie in ihrem mittleren Bereich dünner sind als in zwei einander gegenüber, auf einer durch den mittleren Bereich verlaufenden Gerade liegenden Endbereichen und bevorzugt einen durch ihre mittleren Bereich und senkrecht zu der genannten Gerade angeordneten Bruchspalt aufweisen. Beim Halbieren liegen die genannten Endbereiche der Tablette auf der Unterlage auf, während der mittlere Bereich von der Unterlage beabstandet ist. Die Tablette wird durch Hebelwirkung in zwei Hälften geteilt, indem mit einem Finger auf ihren mittleren Bereich zur Unterlage hin gedrückt wird.

Eine solche Tablette, die bevorzugt eine runde Form hat, ist in der EP-Anmeldung 0 207 888 A1 beschrieben. Die runde Form ist an sich günstig im Hinblick auf die Verarbeitung, insbesondere beim Pressen und beim Versehen mit einem Überzug. Aber bezüglich ihrer Teilbarkeit sind die runden Tabletten den länglichen unterlegen, da beim Halbieren viel Kraft aufgewendet werden muß, weil der Hebelarm relativ kurz und die Bruchfläche relativ groß ist. Eine längliche Tablette dieser Art, bei der die Tabletten beim Teilen mit einer zur Verbindungslinie der Enden senkrechten Linie oder schmalen Fläche auf der Unterlage aufliegen, ist in dem US-Patent 5,061,494 beschrieben. Bedingt durch die Ausgestaltung der Bereiche, mit denen die Tablette auf der Unterlage aufliegt, weist die Tablette eine fast rechteckige Form auf. Die auf der einen Tablettenseite vorhandenen zur Verbindungslinie senkrechten Linien oder schmalen Flächen liegen dabei auf heraustretenden Bereichen an den Tablettenenden, die durch eine konkav zur Tablettenmitte abfallende Senke von einander getrennt sind. Eine andere, längliche Tablette dieser Art ist in dem US-Patent 4,735, 805 beschrieben. Diese Tablette besteht aus einer eliptischen Platte, auf der beidseitig kegelstumpfförmige Erhebungen vorgesehen sind, in die derart eine senkrecht zur Längsachse verlaufende Rinne mit rundem Profil eingebracht ist, daß der jeweils tiefste Punkt der Rinne sich etwa in der geometrischen Mitte der Platte befindet und an den beiden Enden ein einen Scheitelpunkt überquerender Grat verläuft. d.h. die kegelstumpfartigen Erhebungen weisen einen konkaven Einschnitt auf. Die in den beiden US-Patenten beschriebenen Tabletten haben gegenüber der in dem EP-Anmeldung beschriebenen Tablette zwar Vorteile bezüglich der Teilbarkeit, aber Nachteile bei der Herstellung, insbesondere beim Überziehen, was offenbar damit zusammenhängt, daß sie von der runden, gewölbten Form relativ stark abweichen, wobei die in dem US-Patent 4,735,805 beschriebene Tablette außer durch die längliche Form auch noch durch den konkaven Einschnitt von der runden gewölbten Form abweicht. Die letztgenannte Tablette neigt wegen ihrer eliptischen Form außerdem auf den Zuführungsschienen der Verpackungsmaschinen zum Verkanten.

Eine Tablette rationell verpacken oder mit einem Überzug versehen zu können, sind wichtige Kostenkriterien bei der Tablettenherstellung, weil insbesondere das Überziehen mit einem Überzug sehr oft durchgeführt werden muß, um einen schlechten Geschmack oder ein unschönes Aussehen von Tabletten zu überdecken. Voraussetzung für die problemlose Herstellung des Überzugs ist, neben der Überzugsrezeptur und der Qualität der zu überziehenden Tabletten, daß diese im Dragierkessel einwandfrei laufen (rollieren). In der Praxis rollieren nur runde, gewölbte Tabletten einwandfrei. Tabletten, deren Form stark von der runden, gewölbten Form abweicht, wie beispielsweise konventionelle Oblong-Tabletten, rollieren dagegen nicht zufriedenstellend. Diese Oblong-Tabletten, welche die Form einer flachen Platte mit zwei großen Oberflächen, mit zueinander parallelen, geraden, senkrecht zu den großen Oberflächen verlaufenden Längsseiten und mit runden Enden haben, werden beim Dragieren nicht mit einem einheitlichen Überzug versehen. Darüber hinaus verhaken sie sich an der Wand des Dragierkessels untereinander und bilden Strukturen aus, die einem Netz ähnlich sind. Dadurch werden sie an der Kesselwand hochgezogen und brechen dann, weit oberhalb des Kernbettes ab, um in das Kernbett zurückzufallen. Bei grösseren Dragierkesseln ist die Fallhöhe so groß, daß dabei Tabletten zu Bruch gehen können. Auch der Lauf innerhalb des Kernbettes ist eher ein Rutschen als ein Rollieren. Es ist sogar häufig so, daß die Tabletten überhaupt nicht laufen, sondern "durchrutschen". Daher bleibt es nicht aus, daß die Tabletten inhomogen beschichtet werden, was besonders bei farbigen Filmtabletten mit dem bloßen Auge erkennbar ist. Haben dagegen die Tabletten die Eigenschaft zu rollieren, wird die Beschichtung einheitlich. Es ist deshalb die Aufgabe der Erfindung, eine Tablette zur Verfügung zu stellen, die die oben genannten Nachteile nicht besitzt. Insbesondere sollte sie in einem Dragierkessel gut rollieren, einfach herstell- und überziehbar sein und gegebenenfalls teilbar sein, damit sie gerade von älteren oder sehbehinderten Menschen mühelos gehandhabt und halbiert werden kann.

Diese Aufgabe wird mit einer Tablette der eingangs genannten Art gelöst, bei welcher die heraustretenden Bereiche als Ausbauchungen ausgebildet sind.

Die erfindungsgemäße Tablette hat alle Vorteile bezüglich der Teilbarkeit und Greifbarkeit der in den drei genannten Schriften beschriebenen Tabletten. Insbesondere kann die erfindungsgemäße Tablette mühelos gehandhabt und halbiert werden, und ist darüber hinaus einfach herstell- und überziehbar. So wurde gefunden, daß - offenbar dank der Ausbauchungen - die neue Tablettenform im Bett eines Dragierkessels einwandfrei rolliert, d.h. sich wie eine runde, gewölbte Tablette verhält, und sich damit problemlos mit einem einheitlichen Überzug versehen läßt. Das war nicht vorhersehbar. Als weiterer Vorteil kommt hinzu, daß die erfindungsgemäße Tablette im Verhältnis zur Oberfläche ein relativ großes Volumen hat, so daß sie kleinere Abmessungen hat als andere Tabletten mit demselben Volumen. Beim Halbieren der erfindungsgemäßen Tablette haben die Ausbauchungen, die ja konvex geformt sind, gegenüber der in dem US-Patent 4,735,805 beschriebenen konkaven Gestaltung den Vorteil, daß der drückende Finger von allein zum tiefsten Punkt, d.h. zur Tablettenmitte gleitet, wo die Teilung stattfinden soll, was gerade bei älteren Patienten, deren Sehvermögen eingeschränkt ist, hilfreich ist, bzw. die Handhabung durch diese erst ermöglicht. Dieser Handhabungsvorteil ist noch ausgeprägter gegenüber in dem US-Patent 5,061,494 beschriebenen Tabletten, bei denen die Tablettenseite gegen die beim Teilen gedrückt wird, abgesehen von einem Bruchspalt, eben ist.

Eine teilbare Tablette, die ebenfalls heraustretende Bereiche aufweist, welche die Form von Ausbauchungen haben, ist aus dem US-Patent 4,824,677 bekannt. Bei dieser bekannten Tablette treten aber die Ausbauchungen nicht aus der Tablettenunterseite und/oder Tablettenoberseite heraus. Vielmehr treten sie aus dem Tablettensteg heraus, d.h. gegenüber den erfindungsgemäßen Tabletten treten sie in einer um etwa 90° verschiedenen Richtung aus der Tablette heraus. Anders als bei der erfindungsgemäßen Tablette, wird deshalb beim Teilen der bekannten Tablette nicht gegen heraustretende Ausbauchungen gedrückt.

Günstig ist es, wenn die Tablette hantelförmig ist, d.h. eine zur Längsachse der Tablette rotationssymmetrische Form aufweist, weil sie dann in jeder Stellung zur Unterlage durch Fingerdruck in zwei Hälften geteilt werden kann. Eine so geformte Tablette läßt sich auch - unabhängig von Ihrer Stellung zur Unterlage - leicht im mittleren, immer von der Unterlage beabstandeten Bereich aufnehmen.

Eine günstige Ausgestaltung der erfindungsgemäßen Tablette weist eine flache Platte auf, wobei die Ausbauchungen aus den beiden einander gegenüberliegenden großen Plattenflächen herausragen.

Vorteilhaft für die Herstellbarkeit der Tablette ist es, wenn die Platte die Form eines Oblongs mit etwa halbkreisförmigen Endbereichen und zur Längachse parallelen seitlichen Kanten im Mittelbereich der Tablette (1) aufweisen, wobei der Abstand zwischen den zur Längsachse parallelen Kanten bevorzugt gleich, aber gegebenenfalls auch kleiner oder größer als der Durchmesser der Halbkreise an den Enden des Oblongs ist, wobei, wenn der Abstand kleiner ist, die Kreisausschnitte größer als ein Halbkreis sind. Die Presswerkzeuge, um eine solche Tablette zu erzeugen, sind - ähnlich wie diejenigen für konventionelle Oblong-Tabletten - einfach herzustellen und durch die zur Längachse parallelen seitlichen Kanten im Mittelbereich der Tablette ist ihre gute Führung auf Verpackungsmaschinen gewährleistet. Auch neigen die so geformten erfindungsgemäßen Tabletten trotz der oblongförmigen Platte - anders als konventionelle Oblong-Tabletten - beim Dragieren im Dragierkessel nicht dazu, sich untereinander zu verhaken und netzartige Strukturen mit den oben beschriebenen Nachteilen auszubilden. Die so ausgebildete erfindungsgemäße Tablette ist also nicht nur bei der Tablettenherstellung, sondern auch bei der Weiterverarbeitung, d.h. beim Überziehen und/oder Verpacken, sehr vorteilhaft. Es kommt hinzu, daß die erfindungsgemäße Tablette mit oblongförmiger PLatte bei gleichem Volumen kleinere Plattenabmessungen hat als eine konventionelle Oblong-Tablette, welche keine konvexen Ausbauchungen aufweist. Die Tablette wirkt darüber hinaus subjektiv kleiner und damit weniger "bedrohlich" als eine konventionelle Oblong-Tablette mit gleich großer Platte.

Es ist günstig, wenn bei der Tablette mit oblongförmiger Platte die Ausbauchungen mindestens im Bereich der Scheitelpunkte Kugelsegmente bilden. Insbesondere dann, wenn der Kugeldurchmesser der Kugelsegmente großer ist als die Höhe (Ausdehnung senkrecht zu den großen Flächen der Platte) der Tablette, ist die Tablette besonders leicht handhabbar, weil sie sich, wenn sie mit zwei Kugelsegmenten Kontakt zu einer ebenen Unterlage hat, von selbst so auf der Unterlage positioniert, daß die großen Flächen der Platte etwa parallel zur Unterlage ausgerichtet sind, d.h. die für die Teilung günstige Lage einnehmen. Der Kontakt der Kugelsegmente zur Unterlage ergibt sich praktisch von selbst, wenn die größte Ausdehnung (Breite) der großen Flächen senkrecht zur Längsachse der Tablette größer ist als die Höhe der Tablette.

Zur weiteren Erleichterung der Teilbarkeit ist es vorteilhaft, wenn die Tablette in ihrem mittleren Bereich mindestens einen Bruchspalt aufweist, der etwa senkrecht zu der Verbindungslinie zwischen den Endbereichen der Tablette verläuft, mit denen die Tablette beim Teilen auf einer Unterlage aufliegt.

Bei der bevorzugten Ausführungform der erfindungsgemäßen Tablette werden nach dem Teilen die Tablettenhälften in der Regel mit dem halbkreiförmigen Endbereich auf der Unterlage aufliegen, während die Bruchkante schräg nach oben weist. Dadurch lassen sich die Hälften sehr leicht ergreifen und von der Unterlage abheben. Diese günstige Stellung nehmen die Tablettenhälften nur dann nicht in jedem Fall ein, wenn - was leicht zu vermeiden ist - der Schwerpunkt der Tablettenhälften auf der Verbindungslinie zwischen den beiden zu jeder Tablettenhälfte gehörenden Scheitelpunkten liegt.

Bei einer weiteren vorteilhaften Ausgestaltung der Tablette verläuft über den Scheitelpunkt mindestens einer der Ausbauchungen eine Nut, deren Projektion auf eine der großen Flächen der Platte mit der Längsachse der Tablette entweder keinen oder höchstens einen spitzen Winkel bildet. Die Tiefe der Nut kann auch null sein. In einer solchen Nut läßt sich eine Bezeichnung, z.B. das Logo - oder ein Teil davon - des Tablettenherstellers, unterbringen. Bevorzugt verlaufen die Kanten, welche die Nut mit der Oberfläche der Ausbauchung bildet, etwa spiegelsymmetrich zu einer Ebene, welche etwa senkrecht auf der Platte steht. Berührt eine Tablette mit so verlaufenden Nutkanten eine flache Unterlage hat sie einen noch stabileren Stand auf der Unterlage, als wenn sie diese ausschließlich mit der unversehrten Kugeloberfläche berührt.

Auch dadurch wird die Teilbarkeit zusätzlich erleichtert. Ein ähnlich stabiler Stand der Tablette auf der Unterlage wird erreicht, wenn der Scheitelpunkt mindestens einer der Ausbuchtungen von einer zu den großen Flächen der Platte parallelen, kleinen Fläche gebildet wird.

Die erfindungsgemäße Tablette eignet sich insbesondere als pharmazeutische Darreichungsform für beliebige Wirkstoffe. Als Wirkstoff kann jeder beliebige pharmazeutische Wirkstoff eingesetzt werden, beispielsweise Chemotherapeutica, Phytopharmaka, Vitamine, Enzyme, Hormone, etc. Beispielhaft seien als Wirkstoffe Bezafibrat, Clodronsäure, Carvedilol oder Glibenclamid genannt. Die Wirkstoffe können in jeder gewünschten Dosierung enthalten sein, beispielsweise von 0,1 - 100 Gew.-% des Tablettenkerns. Für den Fall, daß sich die Wirkstoffe ohne weitere pharmazeutische Hilfs- oder Trägerstoffe direkt zu Tabletten verpressen lassen, kann die erfindungsgemäße Tablette auch aus dem reinen Wirkstoff bestehen. Üblicherweise werden jedoch weitere Hilfs- oder Trägerstoffe zur Herstellung verwendet, wie beispielsweise Füllmittel, Tablettensprengmittel, Gleit- oder Formentrennmittel, Bindemittel, Adsorptionsmittel, Lösungsverzögerer, Hydrophilierungsmittel, Fließregulierungsmittel, Resorptionsbeschleuniger, Geschmackskorrigentien oder Farbstoffe.

Die Herstellung der Tabletten erfolgt vorzugsweise aus einem Granutlat, das durch Trocken-, Feucht- oder Sprühgranulation der Tablettenbestandteile oder durch Kompaktierung hergestellt wurde.

Weitere vorteilhafte Ausgestaltungen der erfindungsgemäßen Tablette sind in Unteransprüchen offenbart.

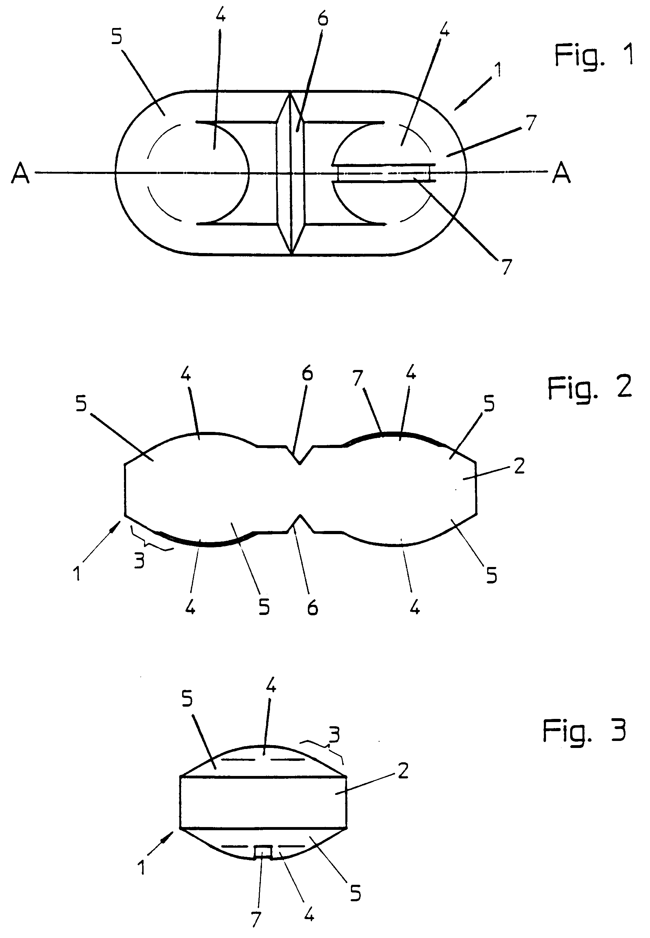

Die Erfindung wird anhand von durch Zeichnungen erläuterten Ausführungsbeispielen beschrieben. Es zeigen Fig. 1 in Aufsicht eine vergrößerte Darstellung einer Ausführungsform der erfindungsgemäßen Tablette, welche auf einer waagrechten Unterlage in einer für die Zweiteilung geeigneten Stellung aufliegt, Fig. 2 einen Längsschnitt durch die in der Fig. 1 gezeigte Tablette entlang der in die Fig.1 eingezeichneten Linie A - - A und Fig. 3 eine Ansicht in Richtung der Längsachse der in den Fig.1 und 2 gezeigten Tablette.

Die durch die Fig. 1 bis 3 veranschaulichte Tablette 1 weist alle wesentlichen und vorteilhaften Merkmale der Erfindung auf. Es sei aber klargestellt, daß, obwohl die anhand der Figuren veranschaulichte Tablette die bevorzugteste Ausführungsform darstellt, auch andere vom Schutzumfang der Ansprüche umfaßte Ausführungsformen der erfindungsgemäßen Tablette aufgrund ihrer wirtschaftlichen Herstellbarkeit und/oder guten Handhabbarkeit vorteilhaft einsetzbar sind.

Fig. 1 zeigt einen Oblong, d.h. ein Gebilde, welches aus einem in zwei Hälften zerschnittenen Kreis und einem Rechteck besteht, das zwischen den Schnittkanten eingefügt ist und dessen den Halbkreisen zugewandte Seiten gleich lang sind wie der Durchmesser des Kreises. Das Oblong bildet die Grundfläche einer Platte 2 (s. Fig. 2 und 3). Die Platte 2 hat drei Symmetrieebenen (im folgenden mit C, D und E bezeichnet), die in den Fig. nicht gezeigt sind. Die Symmetrieebenen C und D stehen senkrecht auf den zueinander parallelen großen Flächen der Platte 2 und die Symmetrieebene E liegt parallel zu den großen Flächen. Die Längsachse der Tablette 1 verläuft in der Schnittlinie der Symmetriebenen C und E. Der in der Fig. 2 gezeigte Längsschnitt fällt mit der Symmetrieebene C zusammen und und der in der Fig. 3 gezeigte Querschnitt verläuft parallel zur Symmetrieebene D. Beide großen Flächen der Platte 2 weisen je zwei Ausbauchungen 3 auf. Die Projektionen der Scheitelpunkte der Ausbauchungen 3 senkrecht auf die Platte 2 fallen mit den Kreismittelpunkten der Halbkreise zusammen.

Im Bereich der Scheitelpunkte werden die Ausbauchungen 3 von Kugelsegmenten 4 gebildet. Den Übergang zwischen den Kugelsegmenten 4 und der Platte 2 bilden zwei Platten 5, deren an die großen Flächen der Platte 2 grenzende Oberflächen mit dem Oblong kongruent sind und die sich zu den Kugelsegmenten 4 hin verjüngen, so daß die Platten 5 im Bereich der halbkreiförmigen Enden stetig in die Kugelsegmente 4 übergehen. Es sei klargestellt, daß die Aufteilung der Tablette in Platten 2 und 5 und Kugelsegmente 4 nur der eindeutigen Beschreibung der Tablette dient. In Wirklichkeit bilden diese Formelemente eine in in einem Preßvorgang erzeugte Einheit.

Der Kugeldurchmesser der Kugelsegmente 4, d.h. der Durchmesser der Kugeln, von denen die Kugelsegmente einen Teil bilden, ist größer als die Höhe der Tablette. Dies macht die Tablette besonders leicht handhabbar, weil sie sich, wenn sie mit zwei Kugelsegmenten Kontakt zu einer ebenen Unterlage hat, von selbst so auf der Unterlage positioniert, daß die großen Flächen der Platte etwa parallel zur Unterlage ausgerichtet sind. Der Kontakt der Kugelsegmente zur Unterlage ergibt sich praktisch von selbst, wenn - was bei der bevorzugten Ausführungsform der Tablette gegeben ist - die größte Ausdehnung (Breite) der großen Flächen senkrecht zur Längsachse der Tablette größer ist als die Höhe der Tablette. Die Platten 5 sind durch symmetrisch zur Symmetrieebene D verlaufende keilförmige Bruchspalte 6 je in zwei gleiche Hälften geteilt.

Eine Nut 7 mit rechteckigem Querschnitt ist spiegelsymmetrisch zur Symmetrieebene C in je eines der Kugelsegmente 4 auf beiden Seiten der Platte 2 eingebracht. Die Nut 7 kann auf Ihrem Boden das Logo - oder einen Teil davon - der Firma tragen, welche die Tablette vertreibt.

Typischerweise ist die Länge des Oblongs etwa 17 und seine Breite etwa 8 mm und die Ausbauchungen 3 haben eine Höhe von je etwa 1,5 mm bei einer Gesamthöhe (Abstand zwischen zwei zur Platte 2 spiegelsymmetrischen Scheitelpunkten der Ausbauchungen) von etwa 5,5 mm. Die Länge der Tablette sollte so bemessen sein, daß der Fingerdruck beim Teilen nicht auf die Scheitelpunkte der Ausbauchungen, sondern auf den Bereich dazwischen wirkt.

Die beschriebene Tablette 1 hat vorteilhafte Eigenschaften. Liegt die Tablette so auf einer flachen Unterlage, daß die großen Flächen der Platte 2 parallel zur Unterlage ausgerichtet sind und die Kugelsegmente 4 auf der einen Plattenseite die Unterlage berühren - eine Stellung, welche die Tablette, wie oben beschrieben, von selbst einnimmt -, so hat der mittlere Bereich der Tablette keinen Kontakt zur Unterlage. Die Tablette läßt sich deshalb dort leicht greifen und abheben. Die beschriebene Stellung der Tablette ist durch die Nut 7 zusätzlich stabilisiert, weil die Kanten, welche die Nut 7 mit der Kugeloberfläche bildet, etwa spiegelsymmetrich zu einer Ebene verlaufen, welche etwa senkrecht auf der Platte 2 steht. Aufgrund des fehlenden Kontakts ihres mittleren Bereichs mit der Unterlage ist die Tablette in der beschriebenen Stellung auch ohne Hilfsmittel mühelos teilbar. Man muß dazu nur auf den mittleren Bereich mit einem Finger von oben Druck ausüben (die Mitte ist die Stelle mit dem maximalen Biegemoment, d.h. die Stelle, wo die Tablette - auch weil sich dort der Bruchspalt befindet - mit der geringsten Kraftanstrengung geteilt werden kann).

Anders als bei den üblichen, runden oder oblongförmigen Tabletten muß dabei die Tablette nicht in die Hand genommen werden. Durch die beiden Kugelsegmente 4 auf der von der Unterlage abgewandtem Seite der Platte 2 wird der Finger, mit dem der Druck ausgeübt wird, automatisch zur Tablettenmitte hingeführt. Daher können auch Personen mit eingeschränktem Sehvermögen die Teilung ohne Hilfsmittel leicht vornehmen. Nach dem Teilen liegen die Tablettenhälften mit den Bruchkanten auf der Unterlage auf, während der halbkreiförmige Endbereich schräg nach oben weist. Dadurch lassen sich die Hälften sehr leicht ergreifen und von der Unterlage abheben. Die Tablette läßt sich außerdem - wie oben bereits erläutert - im Dragierkessel durch Rollieren problemlos mit einem einheitlichen Überzug versehen. Sie verhält sich damit trotz der geraden, zueinander parallel verlaufenden Seitenwänden im mittleren Tablettenbereich wie eine runde, gewölbte Tablette. Aufgrund dieser geraden Seitenwände ist auch das Führen der Tablette in Verpackungsmaschinen ohne Verkanten möglich. Außerdem hat die Tablette ein relativ großes Verhältnis von Volumen zu äußerer Oberfläche und ist dadurch kleiner als - beispielsweise - konventionelle Oblong-Tabletten gleichen Volumens. Darüber hinaus wirkt die Tablette subjektiv kleiner als eine konventionelle Oblong-Tablette, welche dieselben Abmessungen wie die Platte 2 hat.

Es sind auch Ausführungsformen der erfindungsgemäßen Tablette brauchbar, die anders geformt sind als die in den Zeichnungen beschriebenen Tabletten, auch wenn sie nicht alle geschilderten Vorteile aufweisen mögen. Zum Beispiel kann die Tablette andere Abmessungen oder ein anderes Länge:Breite:Höhe-Verhältnis aufweisen. Es ist auch möglich, daß das Rechteck zwischen den halbkreisförmigen Enden schmäler als der Kreisdurchmesser ist, d. h. daß die Tablette Hundekuchenform annimmt. Die Tablette kann auch wie eine Hantel geformt sein. Es sind andererseits auch Formen brauchbar, bei denen entweder die sich zu den Kugelsegmenten 4 hin verjüngenden Platten 5 nicht vorhanden sind oder auf einer Seite der Platte 2 die Ausbauchungen 3 ganz fehlen. Wird eine Tablette mit der letztgenannten Form mit den Ausbauchungen 3 auf eine Unterlage gelegt, ist die gute Teilbarkeit zwar nach wie vor gegeben, aber der Vorteil, daß der Finger automatisch zur Mitte hingeführt wird, entfällt.

## Patentansprüche

1. Längliche, teilbare Tablette, deren Tablettenunterseite und Tablettenoberseite an den beiden Enden je einen derart heraustretenden Bereich aufweisen, daß beim Aufliegen einer der genannten Seiten auf einer flachen Unterlage die heraustretenden Bereiche, aber nicht den Bereich dazwischen, die Unterlage berührt, dadurch gekennzeichnet, daß die heraustretenden Bereiche als Ausbauchungen ausgebildet sind.

2. Tablette nach Anspruch 1, dadurch gekennzeichnet, daß die Tablette hantelförmig ist.

3. Tablette nach Anspruch 1, dadurch gekennzeichnet, daß sie aus einer flachen Platte (2) besteht, wobei die Ausbauchungen (3) aus den beiden einander gegenüberliegenden großen Plattenflächen herausragen.

4. Tablette nach Anspruch 3, dadurch gekennzeichnet, daß die großen Flächen der Platte (2) die Form eines Oblongs mit halbkreisförmigen Endbereichen und zur Längsachse parallelen seitlichen Kanten im Mittelbereich der Tablette (1) aufweisen, wobei der Abstand zwischen den zur Längsachse parallelen Kanten bevorzugt gleich, gegebenenfalls aber auch kleiner oder größer als der Durchmesser der Halbkreise an den Enden des Oblongs ist, wobei, wenn der Abstand kleiner ist, die Kreisausschnitte größer als ein Halbkreis sind.

5. Tablette nach Anspruch 4, dadurch gekennzeichnet, daß die Scheitelpunkte der Ausbauchungen und die Kreismittelpunkte der genannten halbkreisförmigen Endbereiche auf derselben zur Längsachse der Tablette (1) senkrecht verlaufenden Geraden liegen.

6. Tablette nach einem der Ansprüche 1 bis 5, dadurch gekennzeichnet, daß die Ausbauchungen (3) zum mindesten im Bereich der Scheitelpunkte Kugelsegmente (4) bilden.

7. Tablette nach Anspruch 6, dadurch gekennzeichnet, daß den Übergang zwischen den Kugelsegmenten (4) und der Platte (2) sich zu den Kugelsegmenten (4) hin verjüngende, zweite Platten (5) bilden.

8. Tablette nach Anspruch 6 oder 7, dadurch gekennzeichnet, daß der Kugeldurchmesser der Kugelsegmente (4) größer ist als die Höhe der Tablette.

9. Tablette nach einem der Ansprüche 1 bis 8, dadurch gekennzeichnet, daß in ihrem mittleren Bereich mindestens ein Bruchspalt (6) vorgesehen ist, der senkrecht zu der Verbindungslinie zwischen den Endbereichen der Tablette verläuft, mit denen die Tablette beim Teilen auf einer Unterlage aufliegt.

10. Tablette nach einem der Ansprüche 3 bis 9, dadurch gekennzeichnet, daß über den Scheitelpunkt mindestens einer der Ausbauchungen (3) eine Nut (7) verläuft, deren Projektion auf eine der großen Flächen der Platte (2) mit der Längsachse der Tablette keinen oder höchstens einen spitzen Winkel bildet.

11. Tablette nach Anspruch 10, dadurch gekennzeichnet, daß die Kanten, welche die Nut (7) mit der Oberfläche der Ausbauchung (3) bildet, spiegelsymmetrisch zu einer Ebene verlaufen, welche senkrecht auf der Platte (2) steht.

## Claims

1. Divisible oblong tablet in which each of the two ends of the tablet underside and upper side have a zone which extends in such a way that, when one of the said sides is laid on a flat surface, the extending zones but not the intervening zone touch the surface, wherein the extending zones are shaped like bulges.

2. Tablet as claimed in claim 1, wherein the tablet is in the form of a dumb-bell.

3. Tablet as claimed in claim 1, wherein it consists of a flat plate (2) in which bulges (3) extend from both of the large plate areas that are opposite to one another.

4. Tablet as claimed in claim 3, wherein the large areas of the plate (2) are in the shape of an oblong with semi-circular end zones and have lateral edges parallel to the longitudinal axis in the middle zone of the tablet (1), the distance between the edges parallel to the longitudinal axis preferably being the same as, but if desired also smaller or larger, than the diameter of the semicircles at the ends of the oblong whereby if the distance is smaller the circle sectors are larger than a semicircle.

5. Tablet as claimed in claim 4, wherein the apexes of the bulges and the centres of the circles of the said semicircular end zones lie on the same straight line running perpendicular to the longitudinal axis of the tablet (1).

6. Tablet as claimed in one of the claims 1 to 5, wherein the bulges (3) form spherical segments (4) at least in the region of the apexes.

7. Tablet as claimed in claim 6, wherein second plates (5) which taper towards the spherical segments (4) form the transition between the spherical segments (4) and the plate (2).

8. Tablet as claimed in claim 6 or 7, wherein the diameter of the spheres of the spherical segments (4) is larger than the height of the tablet.

9. Tablet as claimed in one of the claims 1 to 8, wherein at least one breaking crevice (6) is provided in their middle zone which runs perpendicular to the connecting line between the end zones of the tablet with which the tablet lies on a support during division.

10. Tablet as claimed in one of the claims 3 to 9, wherein a groove (7) runs over the apex of at least one of the bulges (3) whose projection on one of the large areas of the plate (2) forms either no or at most an acute angle with the longitudinal axis of the tablet.

11. Tablet as claimed in claim 10, wherein the edges which the groove (7) forms with the surface of the bulges (3) are mirror-symmetrical to a plane which is perpendicular to the plate (2).

## Revendications

1. Comprimé oblong sécable, dont la face inférieure et la face supérieure présentent chacune, à leurs deux extrêmités, une zone qui est en saillie de telle façon que lorsqu'on dépose l'une des faces mentionnées sur un support plat, les zones en saillies soient en contact avec le support, mais les zones comprises entre les zones en saillie ne le soient pas, caractérisé par le fait que les zones en saillie ont une conformation bombée.

2. Comprimé selon la revendication 1, caractérisé par le fait que le comprimé est en forme d'haltère.

3. Comprimé selon la revendication 1, caractérisé par le fait qu'il est constitué par une plaque plate (2), les parties bombées (3) faisant saillie sur les deux grandes faces de plaque opposées.

4. Comprimé selon la revendication 3, caractérisé par le fait que les grandes faces de la plaque (2) présentent une forme oblongue, avec des zones d'extrêmité en forme de demi-cercle, et des arêtes latérales parallèles au grand axe dans la zone centrale du comprimé (1), la distance entre les arêtes parallèles au grand axe étant de préférence identique ou éventuellement inférieure ou supérieure au diamètre des demi-cercles aux extrêmités du comprimé oblong, et dans le cas où cette distance est inférieure, les secteurs du cercle étant supérieurs à un demi-cercle.

5. Comprimé selon la revendication 4, caractérisé par le fait que les apex des parties bombées et les centres des cercles dans lesquels s'inscrivent les zones d'extrêmité en forme de demi-cercle mentionnées se trouvent sur la même droite perpendiculaire au grand axe du comprimé (1).

6. Comprimé selon l'une quelconque des revendications 1 à 5, caractérisé par le fait que les parties bombées (3) forment, au moins à proximité de l'apex, des segments sphériques (4).

7. Comprimé selon la revendication 6, caractérisé par le fait que les jonctions entre les segments sphériques (4) et la plaque (2) forment deux plaques (5) s'amenuisant pour former les segments sphériques (4).

8. Comprimé selon la revendication 6 ou 7, caractérisé par le fait que le diamètre de la sphère des segments sphériques (4) est supérieur à la hauteur du comprimé.

9. Comprimé selon l'une quelconque des revendications 1 à 8, caractérisé par le fait que dans sa zone centrale, il est prévu au moins une encoche (6) qui s'étend dans le sens perpendiculaire à la droite reliant les zones d'extrêmité du comprimé par l'intermédiaire desquelles le comprimé repose en partie sur un support.

10. Comprimé selon l'une quelconque des revendications 3 à 9, caractérisé par le fait qu'au travers de l'apex d'au moins une des parties bombées (3) s'étend une rainure (7) dont la projection sur une des grandes faces de la plaque (2) ne forme pas d'angle avec le grand axe du comprimé ou au plus, forme un angle aigü avec celui-ci.

11. Comprimé selon la revendication 10, caractérisé par le fait que les arêtes que forme la rainure (7) avec la surface des parties bombées (3), sont symétriques par rapport à un plan qui est perpendiculaire à la plaque (2).
